# EUROPEAN PATENT SPECIFICATION

(11) **EP 2 749 298 B1**
(45) Date of publication and mention of the grant of the patent: **06.04.2016**
(21) Application number: 12382535.8
(22) Date of filing: 26.12.2012
(51) Int. Cl.: A61L 27/00, C07D 249/04, A61K 31/4192

(54) **A 1,4,5-TRISUBSTITUTED1,2,3-TRIAZOLE MIMETIC OF RGD and/or OGP10-14, PROCESS TO OBTAIN IT AND USES THEREOF**
1,4,5-trisubstituiertes 1,2,3-Triazolmimetikum von RGD bzw. OGP10-14, Herstellungsverfahren und Verwendungen dafür
Mimétique de 1,2,3-triazole 1,4,5-trisubstituées de RGD et/ou OGP10-14, procédé d'obtention et utilisations associées

(43) Date of publication of application: 02.07.2014
(73) Proprietor: Universidad Del Pais Vasco-Euskal Herriko Unibertsitatea, 48170 Zamudio (Vizcaya) (ES); FUNDACIÓN TECNALIA RESEARCH & INNOVATION, 20009 San Sebastian (Guipuzcoa) (ES); Centro de Investigación Biomedica en Red en Bioingenieria, Biomateriales y Nanomedicina, 50018 Zaragoza (ES)
(72) Inventor: Aizpurua Iparraguirre, Jesús, Mª, 48170 Zamudio (Vizcaya) (ES); Sagartzazu Aizpurua, Maialen, 48170 Zamudio(Vizcaya) (ES); Braceras Izaguirre, Iñigo, 20009 Donostia-San-Sebastian (ES); Azpiroz Dorronsoro, Francisco, Javier, 20009 Donostia-San-Sebastian (ES); Oyarbide Vicuna, Joseba, 50018 Zaragoza (ES); Fernandez Oyon, Xavier, 48170 Zamudio (Vizcaya) (ES)
(74) Representative: Carpintero Lopez, Francisco

(56) References cited:
- WO-A1-97/32594
- SANDER S. VAN BERKEL ET AL: "Application of Metal-Free Triazole Formation in the Synthesis of Cyclic RGD-DTPA Conjugates", CHEMBIOCHEM, vol. 9, no. 11, 21 July 2008 (2008-07-21), pages 1805-1815, XP055056048, ISSN: 1439-4227, DOI: 10.1002/cbic.200800074

## Description

### FIELD OF THE INVENTION

The present invention relates to nonpeptide mimetics of RGD (Arg-Gly-Asp) and/or, OGP₁₀₋₁₄ (Tyr-Gly-Phe-Gly-Gly), osteogenic compounds, containing a central 1,4,5-trisubstituted 1,2,3-triazole core and a reactive appendage appropriate to form covalent "click" bonds on the surface of materials functionalized with reactive groups including: azide, terminal alkyne, cyclooctalkyne, thiol, maleimide or thiolacid groups and to a process for the preparation of RGD and OGP₁₀₋₁₄ mimetics containing a 1,4,5-trisubstituted 1,2,3-triazole core.

These nonpeptide mimetics are particularly useful for medical devices, including endosseous implants, or tissue engineering scaffold or cell culture matrix, suitable for the replacement or regeneration of human and animal organs.

### BACKGROUND ART

Some natural short peptides occurring in the extra cellular matrix (ECM) or in serum are useful to enhance the adhesion, viability and proliferation of osteoblast cells.

RGD occurs in several ECM proteins, including vitronectin, fibronectin and osteopontin. The technological interest in RGD tripeptide analogues in applications related to osteogenesis stems from their ability to regulate the out-in signaling of ECM with integrin receptors in osteoblast cells (Kantlehner M et al. ChemBioChem 2000 1: 107-114 "Surface coating with cyclic RGD peptides stimulates osteoblast adhesion and proliferation as well as bone formation"). Using RGD analogues may be beneficial to regulate bone remodeling by promoting the recruitment, attachment and differentiation of osteoblasts and osteoclasts, as demonstrated for example in osteoporosis therapy (Chen, W. et al in Biotechnol. Lett. 2005 27: 41-48 "Bone loss induced by ovariectomy in rats is prevents by gene transfer of parathyroid hormone or an Arg-Gly-Asp-containing peptide").
The osteogenic growth peptide (OGP) is a short naturally occurring 14-mer growth factor peptide found in serum. As a soluble peptide, OGP regulates proliferation, differentiation, and matrix mineralization in osteoblast lineage cells. Studies have demonstrated sensitivity of osteoblast lineage cells to changes in exogenous concentrations of OGP. It has also been shown in the art that only the OGP₁₀₋₁₄ (Tyr-Gly-Phe-Gly-Gly) pentasegment is essential for the osteogenic activity of OGP (Chen, Y.-Ch. et al. J. Med. Chem. 2002 45: 1624-1632 "Bioactive pseudopeptidic analogues and cyclostereoisomers of osteogenic growth peptide C-terminal pentapeptide, OGP10-14").

Attaching RGD peptide analogues to the surface of bone or endosseous implants has shown to be beneficial to improve osteoblast cell adhesion (see for example Kessler H et al. Biomaterials 2003 24: 4385-4415 "RGD modified polymers: biomaterials for stimulated cell adhesion and beyond"; see also: Biltresse S. et al. Biomaterials 2005 26: 4576-4587 "Cell adhesive PET membranes by surface grafting of RGD peptidomimetics"). On the other hand, the effectiveness of immobilized OGP to increase osteoblast cell densities and fasten their proliferation rate on OGP-stained materials has been proven "in vitro" (Moore, N. M. et al. Biomaterials 2010 31: 1604-1611 "The use of immobilized osteogenic growth peptide on gradient substrates synthesized via click chemistry to enhance MC3T3-E1 osteoblast proliferation").

Natural RGD and OGP peptides, their pseudopeptide cyclic analogues or their amidic surrogates are prone to suffer a fast deactivation by proteolytic cleavage "in vivo", leading to lowered serum concentrations in systemic applications or inactive surfaces when grafted to biomaterials. Several RGD analogues known in the art may partially overcome this drawback, including some 1,4-disubstituted 1,2,3-triazole compounds (see Trabocchi, A. et al. J. Med. Chem. 2010, 53: 7119-7128 "Click-Chemistry-derived triazole ligands of Arginine-Glycine-Aspartate (RGD) integrins with a broad capacity to inhibit adhesion cells and both in vitro and in vivo angiogenesis" and Ni, M. H. et al Lett. Drug Design & Discovery 2011, 8: 401-405 "Novel RGD peptidomimetics embedding 1,2,3-triazole as central scaffold; synthesis and αvβ3 integrin affinity"). Conversely, some pseudopeptide OGP mimetics are also known in the art (see Bab, I. et al WO9732594A1 "Synthetic peptides and pseudopeptides having osteogenic activity and pharmaceutical compositions containing the same").

However, none of these RGD or OGP10-14 nonpeptide mimetics are amenable to embodiments capable to give further "click" attachment to the surface of biomaterials.

Thus, from what is known in the art, it is derived that the development of a RGD or an OGP10-14 nonpeptide mimetic material capable to attach to the surface of biomaterials is still of great interest.

### SUMMARY OF THE INVENTION

Inventors have developed 1,4,5-trisubstituted 1,2,3-triazole mimetics of RGD and/or OGP₁₀₋₁₄ that allow to conduct in a single chemical operation the "click" bonding on the surface of a material. The peptidomimetics bound in this way are stable and do not suffer degradation reactions under physiological conditions.

Therefore an aspect of the invention relates to a 1,4,5-trisubstituted 1,2,3-triazole mimetic of RGD and/or OGP₁₀₋₁₄ of formula (I): 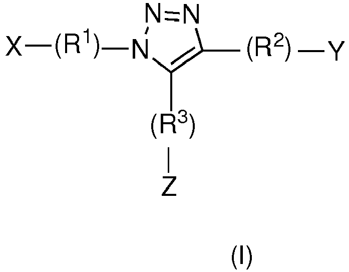 wherein:
R¹ is a biradical selected from the group consisting of C₁₋₂₀ alkylene; in which 0, 1, 2, 3, 4, 5 or 6 -CH₂- groups are optionally replaced by groups selected from -O-, -S-, -C(O)O-, -C(O)NH-, -C(O)N(C₁₋₄alkyl)-, -NHC(O)NH-,-NHC(O)O-;
R² is a biradical independently selected from the group consisting of: C₁₋₆ alkylene; in which 0, 1, 2 or 3 -CH₂- groups are optionally replaced by groups selected from -O- and -S-; optionally substituted with one or more groups selected from C₁₋₄ alkyl, phenyl, C₆₋₁₀ aryl; and
R³ is a biradical selected from the group consisting of: C₁₋₆ alkylene; optionally containing one or more -C=C- bonds; optionally containing -C=C- bonds; in which 0, 1, 2 or 3 -CH₂- groups are optionally replaced by groups selected from -O- and -S-; optionally substituted with one or more groups selected from C₁₋₄ alkyl, phenyl, -F, -Cl, -OH, -O(C₁₋₄alkyl), -S(C₁₋₄alkyl), -SO₂Ph, - CN, -NO₂, -CO(C₁₋₄alkyl), -CO₂H, -CO₂(C₁₋₄alkyl), -CONH₂, -CONH(C₁₋₄alkyl), -CON(C₁₋₄alkyl)₂; and
X is a group selected from the group consisting of: azide, N-maleimide, N-maleimide-furan cycloadduct, thiol, thio acid, sulfonylazide, ethynyl, iodoethynyl and an activated cyclooctynyl group represented by the formulae: 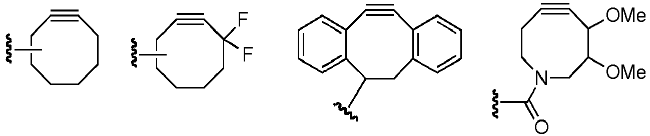
Y is a group selected from the group consisting of: -NHC(=NH)NH₂, C₆H₄-OH
Z is a group selected from the group consisting of: -CO₂H, Ph
   and
when Y is -NHC(=NH)NH₂, Z is -CO₂H and when Y is C₆H₄-OH, Z is Ph.

Another aspect of the invention is a process for preparing the compounds as defined above wherein Z is -CO₂H and Y is -NHC(=NH)NH₂, which comprises:
a) reacting an azide of formula Q-(R¹)-N₃ (II) with an alkyne of formula T-C≡C-(R²)-NH-PG (protecting group) (III) to obtain a triazole of formula (IV);
b) reacting a triazole of the formula (IV) with a compound of formula S-(R³)-CO₂R^{a} (V) or an alkene of formula H₂C=CH-CO₂R^{a} (VI), to provide a compound of formula (VII);
c) replacing the Q group in the compounds of formula (VII) by a group X to obtain a compound of formula VIII; and d) removing the protecting group (PG) in compounds of the formula (VIII) and e) reacting the intermediate amines with H₂NC(=NH)SO₃H. 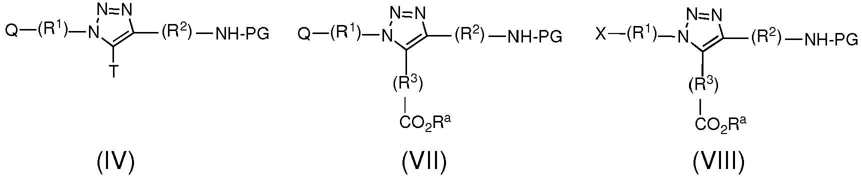 wherein:
   R¹, R², R³ and X are as defined above; R^{a} is H, methyl, ethyl, tert-butyl or benzyl; T is H or I; Q is HO, methanesulfonyloxy, p-toluenesulfonyloxy, 2-nitrobenzenesulfonyloxy, 4-nitrobenzenesulfonyloxy, trifluoromethanesufonyloxy, Cl, Br or I; PG is H, tert-butoxycarbonyl, allyloxycarbonyl or benzyloxycarbonyl; S in step b) is B(OH)₂, methanesulfonyloxy, p-toluenesulfonyloxy, 2-nitrobenzenesulfonyloxy, 4-nitrobenzenesulfonyloxy, trifluoromethanesufonyloxy, Cl, Br or I.

Another aspect of the invention is a process for preparing the compound as defined above wherein, Y is C₆H₄-OH and Z is Ph, which comprises:
a) reacting an azide of formula Q-(R¹)-N₃ (II) with an alkyne of formula T-C≡C-(R²)-( C₆H₄OH)-PG (IX) to obtain a triazole of formula (X),
b) reacting a triazole of the formula (X) with a compound of formula S-(R³)-Ph (XI), to provide a compound of formula (XII),
c) replacing the Q group in the compounds of formula (XII) by a group X, and
d) removing the protecting group PG. 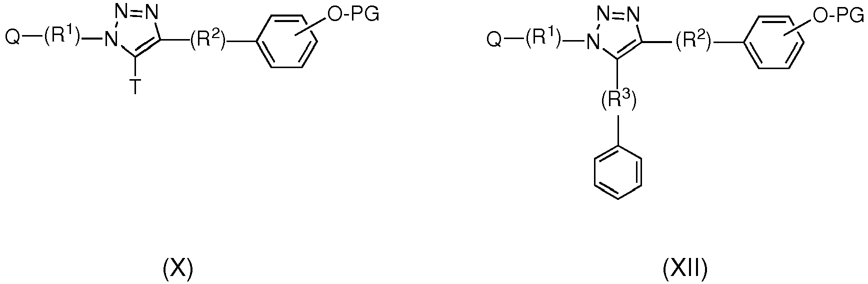 wherein:
   R¹, R², R³ and X are as defined above; T is H or I; Q is HO, methanesulfonyloxy, p-toluenesulfonyloxy, 2-nitrobenzenesulfonyloxy, 4-nitrobenzenesulfonyloxy, trifluoromethanesufonyloxy, Cl, Br or I; PG is H, tert-butoxycarbonyl, allyloxycarbonyl or benzyloxycarbonyl; S in step b) is B(OH)₂; PG is Si(i-Pr)₃.

The appendage group X of the peptidomimetic is useful to bind to different materials when their surface is functionalized with reactive groups such as azide, sulfonylazide, terminal alkyne, terminal iodoalkyne, cycloalkyne, N-maleimide, N-maleimide-furan cycloadduct, thiol or thio acid. As a result of such reactions, a material-W-peptidomimetic adduct is formed, which contains at least one of such W groups:

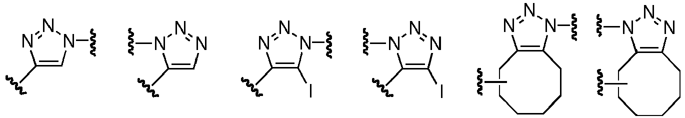

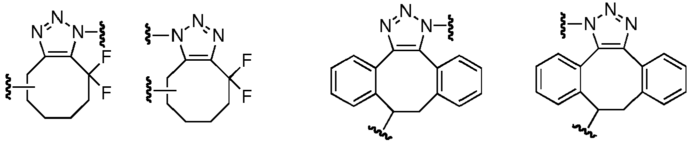

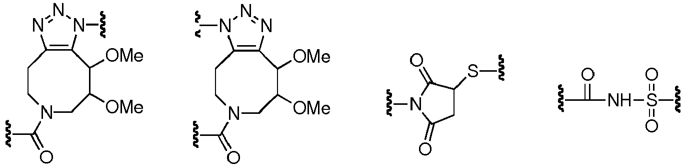

Therefore another aspect of the invention relates to a material with the surface chemically modified wherein the X group of the 1,4,5-trisubstituted 1,2,3-triazole mimetic of RGD and/or OGP₁₀₋₁₄ according to the 1,4,5-trisubstituted 1,2,3-triazole mimetic of RGD and/or OGP₁₀₋₁₄ of formula (I) as defined above is replaced by a W the group as defined above..

Among them, (Arg-Gly-Asp) tripeptide and OGP₁₀₋₁₄ (Tyr-Gly-Phe-Gly-Gly) pentapeptide have potential interest for "in vitro" tissue engineering and diagnosis technologies, or to improve the osteogenic properties of implants in human and animal therapy "in vivo".
Thus another aspect of the invention relates to a medical device, made from the material described above. It can preferentially be an endosseous implant, or tissue engineering scaffold or cell culture matrices.

### BRIEF DESCRIPTION OF THE DRAWINGS

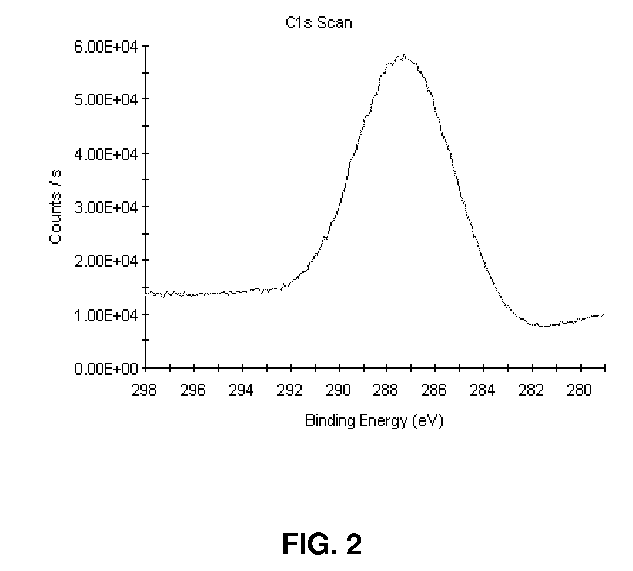

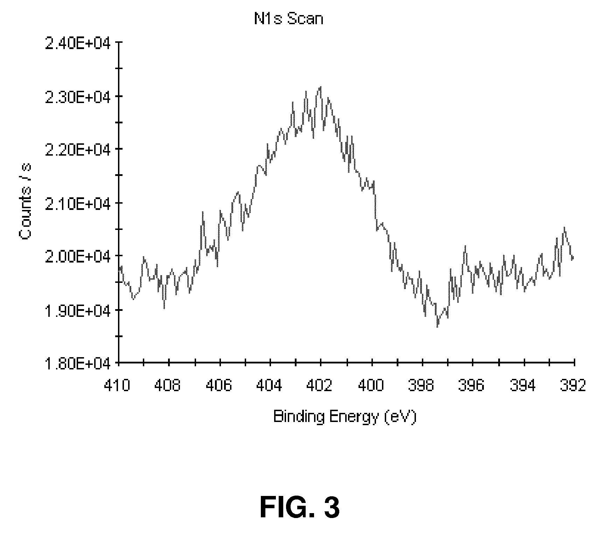

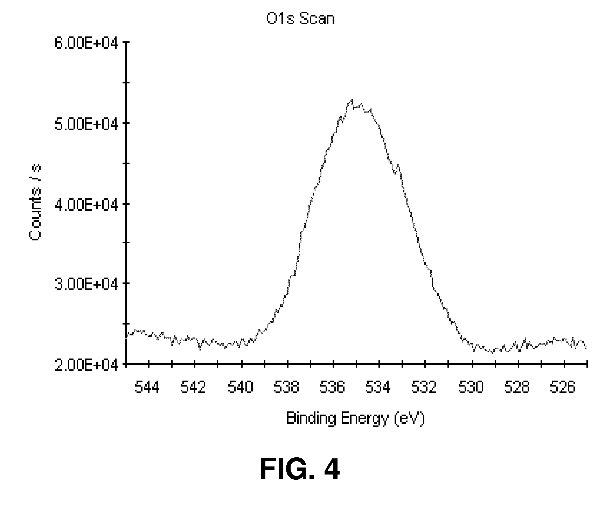

Figure 1 shows the X-ray photoelectron spectroscopy (XPS) spectrum (with three spectra magnifications of C1s, N1s and O1s peaks, Figures 2-4) for a compact PEEK sample modified at the surface with a mixture of RGD and OGP mimetics, as described in example 18.
Figure 2 shows a Figure 1 spectrum magnification of C1s peak.
Figure 3 shows a Figure 1 spectrum magnification of N1s peak.
Figure 4 shows a Figure 1 spectrum magnification of 01 s peak.

### DETAILED DESCRIPTION OF THE INVENTION

As mentioned above, an aspect of the present invention relates to 1,4,5-trisubstituted 1,2,3-triazole mimetics of RGD and/or OGP₁₀₋₁₄ of formula (I) as defined above.

As used herein, the term "alkyl" includes both saturated straight chain and branched hydrocarbon substituents. Preferably, C₁₋₂₀ alkyl groups, more preferably C₁₋₆ alkyl groups. Particularly preferred alkyl groups include, for example, methyl, ethyl, propyl, isopropyl, butyl, isobutyl, tert-butyl, pentyl and hexyl.

As used herein, the term "alkylene" includes biradical hydrocarbon saturated straight chains and branched chains attaching simultaneously two molecular fragments or functional groups. Preferably, C₁₋₂₀ alkylene groups, more preferably C₁₋₆ alkylene groups. Particularly preferred alkylene groups include, for example, methylene, ethylene, propylene and butylene.

As used herein, the term "aryl" includes substituted or unsubstituted single-ring aromatic groups in which each atom of the ring is carbon. Preferably the ring is a 5- to 7-membered ring, more preferably a 6-membered ring.
In a preferred embodiment R¹ is selected from the group consisting of - (CH₂CH₂O)rCH₂CH₂-; where r is an integer between 0 and 8, R² is selected from the group consisting of -CH₂OCH₂CH₂- or -CH₂OCH₂-, and R³ is selected from the group consisting of -CH₂CH₂- or -CH=CH-.

Particularly in the compound of formula (I), Y is -NHC(=NH)NH₂ and Z is - CO₂H. In other particular embodiment, Y is C₆H₄-OH and Z is Ph. Preferably Y is p-C₆H₄-OH and Z is Ph.

Other aspect of the invention relates to a material with the surface chemically modified wherein the X group of the 1,4,5-trisubstituted 1,2,3-triazole mimetic of RGD and/or OGP₁₀₋₁₄ according to the 1,4,5-trisubstituted 1,2,3-triazole mimetic of RGD and/or OGP₁₀₋₁₄ of formula (I) as defined above is replaced by a W the group as defined above. Preferably the material with the surface chemically modified wherein in the 1,4,5-trisubstituted 1,2,3-triazole mimetic Y is -NHC(=NH)NH₂ and Z is -CO₂H. Preferably the surface chemically modified wherein in the 1,4,5-trisubstituted 1,2,3-triazole mimetic Y is p-C₆H₄-OH and Z is Ph.

In a preferred embodiment the material is metals, metallic alloys, polymers, ceramics or composites. In particular, those materials that comply with the ISO 10993 standards for evaluating the biocompatibility. Examples of materials are; Ti, Ti alloys, polyethylene, polypropylene, PET, polyamide, polyester, polyurethanes, silicones or PAEK. More preferably the material is PAEK.

Finally the invention refers to a medical device wherein the device is made from the material as defined above.

As used herein the term "medical device" means any instrument, apparatus, appliance, material or other article, whether used alone or in combination, intended to be used for the purpose of:
- diagnosis, prevention, monitoring, treatment or alleviation of disease,
- diagnosis, monitoring, treatment, alleviation of or compensation for an injury or handicap,
- investigation, replacement or modification of the anatomy or of a physiological process.

As used herein the term "medical device" includes stents, stent grafts, catheters, guide wires, balloons, filters (e.g., vena cava filters), vascular grafts, intraluminal paving systems, pacemakers, electrodes, leads, defibrillators, joint and bone implants, spinal implants, access ports, intra-aortic balloon pumps, heart valves, sutures, artificial hearts, neurological stimulators, cochlear implants, retinal implants, and other devices that can be used in connection with therapeutic coatings, prosthetic bone implant, endooseous implant, scaffold for bone tissue regeneration. Such medical devices are implanted or otherwise used in body structures, cavities, or lumens such as the vasculature, gastrointestinal tract, abdomen, peritoneum, airways, esophagus, trachea, colon, rectum, biliary tract, urinary tract, prostate, brain, spine, lung, liver, heart, skeletal muscle, kidney, bladder, intestines, stomach, pancreas, ovary, uterus, cartilage, eye, bone, joints, and the like. Preferably medical device is an implant or cell culture matrix. More preferably endooseous implant or tissue engineering scaffold or cell culture matrices.

In a preferred embodiment the invention refers a medical device wherein the medical device is an endosseous implant, or tissue engineering scaffold or cell culture matrix, suitable for the replacement of human and animal organs.

### EXAMPLES

Acronyms of reagents, solvents or techniques used are defined as follows:
Boc: tert-Butoxycarbonylamino group,
Dansyl: 5-(Dimethylamino)naphthalene-1-sulfonyl group,
DIPEA: Diisopropyletylamine,
DMF: N,N-Dimethylformamide,
NBS: N-Bromosuccinimide,
NMR: Nuclear Magnetic Resonance,
OGP: Osteogenic Growth Peptide. For the purpose of this invention refers to structures of formula (I), wherein Y is p-C₆H₄-OH and Z is Ph.
PEEK: Poly-ether ether ketone,
RGD: Arginine-Glycine-Aspartic acid. For the purpose of this invention refers to structures of formula (I), wherein Y is -NHC(=NH)NH₂ and Z is -CO₂H
THF: Tetrahydrofuran,
TLC: Thin Layer Chromatography.

The following examples are provided for illustrative means, and are not meant to be limiting of the present invention.

### EXAMPLE 1: Compound (IV): R¹= -CH₂CH₂-; R²= -CH₂OCH₂CH₂-;Q= OH; PG= Boc; T= I 4-(4-tert-Butoxycarbonylamino-2-oxa-butyl)-1-(2-hydroxyethyl)-5-iodo-1,2,3-triazole

To a stirred solution of CuI (218 mg, 1.15 mmol) in dried CH₃CN, NBS (223 mg, 1.25 mmol), 2-(*tert*-butoxycarbonylamino)ethyl propargyl ether (208 mg, 1.04 mmol), 2-azidoethanol (100 mg, 1.15 mmol) and DIPEA (200 µl, 1.15 mmol) were added. The mixture was stirred at room temperature for two hours. The solvent was evaporated, the residue was dissolved in CH₂Cl₂, washed with 10% aqueous Na₂S₂O₃ and the organic phase was dried (MgSO₄) and evaporated. The product was purified by column chromatography (silica gel; EtOAc/hexanes 1:1). Yield: 300 mg (70%). ¹H NMR (500 MHz, CDCl₃) δ 4.63 (s, 2H), 4.54 - 4.48 (t, *J* = 4.9, 2H), 4.18 (t, *J* = 5.0, 2H), 3.62 (t, *J* = 5.1, 2H), 3.34 (t, *J* = 5.0, 2H), 1.46 (s, 9H).

### EXAMPLE 2: Compound (VII): R¹= R³= -CH₂CH₂-; R²= -CH₂OCH₂CH₂-; R^{a}= CH₃; Q= OH; PG= Boc 4-(4-tert-Butoxycarbonylamino-2-oxa-butyl)-1-(2-hydroxyethyl)-5-(2-methoxycarbonyl-ethyl)-1,2,3-triazole:

A suspension of 4-(4-tert-butoxycarbonylamino-2-oxa-butyl)-1-(2-hydroxyethyl)-5-iodo-1,2,3-triazole (500 mg, 1.21 mmol), Pd(OAc)₂ (27,2 mg, 0.121 mmol) and NaHCO₃ (254,7 mg, 3,03 mmol) in anhydrous DMF (5 mL) was prepared in a flame-dried flask under nitrogen atmosphere. Methyl acrylate (273.1 µL, 3,03 mmol) was added and the mixture was stirred at 85ºC overnight. The solvent was evaporated and the product was purified by column chromatography (silica gel, EtOAc/hexanes 1:1). This intermediate product (355 mg, 0.96 mmol) was dissolved in dry MeOH and ammonium formate (302.2 mg, 4.8 mmol) and 10%-Pd-C (107.8 mg, 0.096 mmol) were added. The mixture was refluxed overnight. The product was purified by filtration over celite. Yield: 330 mg (74%). ¹H NMR (500 MHz, CDCl₃): δ 4.55 (s, 2H), 4.40 (t, *J* = 4.7, 2H), 4.06 (t, *J* = 4.7, 2H), 3.64 (s, 3H), 3.54 (t, *J* = 5.1, 2H), 3.26 (s, 2H), 3.05 (t, *J* = *7*.3, 2H), 2.68 (t, *J* = 7.4, 2H), 1.41 (s, 9H).

### EXAMPLE 3: Compound VIII): R¹= R³= -CH₂CH₂-; R²= -CH₂OCH₂CH₂-; R^{a}= CH₃; X= N₃; PG= Boc 1-(2-Azidoethyl)-4-(4-tert-butoxycarbonylamino-2-oxa-butyl)-5-(2-carboxethyl)-1,2,3-triazole

To a stirred solution of 4-(4-tert-butoxycarbonylamino-2-oxa-butyl)-1-(2-hydroxyethyl)-5-(2-methoxycarbonylethyl)-1,2,3-triazole (135 mg, 0.36 mmol) prepared in Example 2, cooled to 0ºC in dried CH₂Cl₂ was added triphenyl phosphine (190.2 mg, 0.73 mmol) and NBS (129.0 mg, 0.73 mmol). The mixture was stirred over one hour. The solvent was evaporated and the crude product was dissolved in dried DMF. Then, NaN₃ (94.3 mg, 1.45 mmol) and Nal (54.3 mg, 0.36 mmol) were added and the mixture was stirred at room temperature over 48 hours. After evaporation of the solvent, the crude product was dissolved in THF/H₂O (1:1) and LiOH·H₂O (151. 9mg, 3.62 mmol) was added and the mixture was stirred for 8 hours. Then, the solvent was evaporated and the product was purified by acid and basic extraction with CH₂Cl₂. Yield: 143.9 mg (70%). ¹H NMR (500 MHz, CD₃OD) δ 4.64 (s, 2H), 4.57 (t, *J* = 5.5, 2H), 3.88 (t, *J* = 5.5, 2H), 3.54 (t, *J* = 5*.*5, 2H), 3.25 (t, *J* = 5.3, 2H), 3.12 (t, *J* = 7.3, 2H), 2.73 (t, *J* = 7.2, 2H), 1.45 (s, 9H).

### EXAMPLE 4: Compound (VIII): R¹= -[CH₂CH₂O]₃CH₂CH₂HN(CO)OCH₂CH₂-; R²= -CH₂OCH₂CH₂-; R³= -CH₂CH₂-; R^{a}= CH₃; X= N₃; PG= Boc 1-(16-Azido-5-aza-3,8,11,14-tetraoxa-4-oxohexadecyl)-4-(4-tert-butoxycarbonylamino-2-oxa-butyl)-5-(2-methoxycarbonylethyl)-1,2,3-triazole

In a flame-dried flask, 4-(4-tert-butoxycarbonylamino-2-oxa-butyl)-1-(2-hydroxyethyl)-5-(2-methoxycarbonyl-ethyl)-1,2,3-triazole (0.27 mmol, 100 mg), prepared as described in Example 2, was dissolved in dry THF (2 mL) under nitrogen atmosphere and after addition of DIPEA (0.54 mmol, 96 µL), the mixture was cooled to 0ºC. Subsequently, a solution of triphosgene (0.17 mmol, 49 mg) was added dropwise and then the mixture was allowed to reach the room temperature during 30 min. The suspension was filtered through a celite pad and the solvent was evaporated under pressure to obtain the intermediate chloroformate, which was immediately dissolved in dry CH₂Cl₂ (2 mL). DIPEA (0.54 mmol, 93 µL) and 8-azido-3,6-dioxaoctylamine (0.27 mmol, 60 mg) were added and the mixture was kept stirring overnight. The product was purified by column chromatography using CH₂Cl₂/MeOH 90/10 as eluent. Yield: 85 mg (51%). IR (cm⁻¹): 3339, 2869, 2102 (N₃), 1704 (C=O), 1522 (tri). ¹H NMR (500 MHz, CDCl₃) δ 4.61 (s, 2H, OC*H*₂C=Cₜᵣᵢ), 4.56 (t, *J=* 5.1, 2H, N₁ₜᵣᵢC*H*₂CH₂), 4.49 (t, *J* = 5.0, 2H, N₁ₜᵣᵢCH₂C*H*₂), 3.67 (s, 3H, COOC*H*₃), 3.67-3.51 (m, 14H, OC*H*₂CH₂NHBoc and OC*H*₂CH₂O), 3.40 - 3.28 (m, 6H, OCH₂C*H*₂NHBoc, OCH₂C*H*₂NHCOO and OCH₂C*H*₂N₃), 3.04 (t, *J* = 7.6, 2H, C*H*₂CH₂CO₂CH₃), 2.70 (t, *J=* 7.2, 2H, CH₂C*H*₂CO₂CH₃), 1.43 (s, 9H, ^{t}Bu).

### EXAMPLE 5: Compound (I): R¹= R³= -CH₂CH₂-; R²= -CH₂OCH₂CH₂-; X= N₃; Y= -NHC(=NH)NH₂; Z= CO₂H 1-(2-Azidoethyl)-5-(2-carboxyethyl)-4-(4-N-guanidyl-2-oxa-butyl)-1,2,3-triazole

A 6M HCl solution in dioxane (2 mL, 12 mmol) was added to 1-(2-azidoethyl)-4-(4-tert-butoxycarbonylamino-2-oxa-butyl)-5-(2-carboxyethyl)-1,2,3-triazole (80 mg, 0.21 mmol) and the mixture was stirred at room temperature over 1 h. Then, the solvent was evaporated, the crude amine hydrochloride was dissolved in methanol (5 mL) and sodium bicarbonate was added until pH=7. Amino(imino)methanesulfonic acid (31.0 mg, 0.25 mmol) was added, the mixture was stirred at room temperature over 1 h. and it was evaporated to dryness. The residue was extracted with MeOH (3 x 5 mL) and the solution was evaporated at reduced pressure. Yield (90 %). ¹H NMR (500 MHz, D₂O): δ 4.62 (s, 1 H), 4.46 (t, *J* = 5.0, 1 H), 3.97 (t, *J* = 5.0, 1 H), 3.68 (t, *J* = 4.8, 1 H), 3.38 - 3.33 (m, 1 H), 3.00 (t, *J* = 7.6, 1 H), 2.43 (t, *J* = 7.6, 1 H).

### EXAMPLE 6: Compound (I): R¹= -[CH₂CH₂O]₃CH₂CH₂HN(CO)OCH₂CH₂-; R²= -CH₂OCH₂CH₂-; R³= -CH₂CH₂-; X= N₃ Y= -NHC(=NH)NH₂; Z= CO₂H 1-(16-Azido-5-aza-3,8,11,14-tetraoxa-4-oxohexadecyl)-5-(2-carboxyethyl)-4-(4-N-guanidyl-2-oxa-butyl)-1,2,3-triazole

A 4M HCl solution in dioxane (2 mL, 8 mmol) was added to 1-(16-azido-5-aza-3,8,11,14-tetraoxa-4-oxohexadecyl)-4-(4-tert-butoxycarbonylamino-2-oxa-butyl)-5-(2-methoxycarbonylethyl)-1,2,3-triazole (75 mg, 0.14 mmol) and the mixture was stirred at room temperature over 2 h. Then, the solvent was evaporated, the crude amine hydrochloride (45 mg, 0.08 mmol) was dissolved in methanol (5 mL) and potassium carbonate was added until slightly basic pH. Amino(imino)methanesulfonic acid (11 mg, 0.09 mmol) was added, the mixture was stirred at room temperature over 1 h. Lithium hydroxide (4.0 mg, 0.09 mmol) was added and the solution was stirred for 4h. Upon completion, the solids were filtered off with MeOH (3 x 5 mL), the solution was evaporated at reduced pressure and the resulting crude product was purified by preparative reverse phase HPLC (C18 column, MeCN:H₂O 80:20). Yield (75 %). ¹H NMR (500 MHz, D₂O) δ 4.63 (m, 4H, OC*H*₂C=Cₜᵣᵢ and N₁ₜᵣᵢC*H*₂CH₂), 4.46 (m, 2H, N₁ₜᵣᵢCH₂C*H*₂), 3.70-3.60 (m, 12H), 3.50 (m, 2H, OC*H*₂CH₂NHCOO), 3.44 (t, *J* = 4.2, 2H, OCH₂C*H*₂N₃), 3.36 (t, *J* = 4.7, 2H, OCH₂C*H*₂NHCOO), 3.18 (m, 2H, CH₂C*H*₂guanidine), 3.02 (t, *J* = 7.4, 2H, C*H*₂CH₂CO₂H), 2.45 (t, *J* = 7.5, 2H, CH₂C*H*₂CO₂H).

### EXAMPLE 7: Compound (X): R¹= -CH₂CH₂-; R²= -CH₂OCH₂-; Q= OH; T= I; PG= Si(ⁱPr)₃ 1-(2-Hydroxyethyl)-5-iodo-4-[2-oxa-3-(4-triisopropylsilyloxyphenyl)-proyl]-1,2,3-triazole

To a stirred solution of CuI (401 mg, 2.11 mmol) in dried CH₃CN, NBS (409 mg, 2.30 mmol), 4-triisopropylsilyloxy-benzyl propargyl ether (610 mg, 1.92 mmol), 2-azidoethanol (100 mg, 1.15 mmol) and DIPEA (367 µl, 2.11 mmol) were added. The mixture was stirred at room temperature for two hours. The solvent was evaporated, the residue was dissolved in CH₂Cl₂, washed with 10% aqueous Na₂S₂O₃ and the organic phase was dried (MgSO₄) and evaporated. The product was purified by column chromatography (silica gel; EtOAc/hexanes 1:1). Yield: 315 mg (31%). ¹H NMR (500 MHz, CDCl₃) δ 7.26 (d, *J =* 8.0, 2H), 6.88 (d, *J* = 8.1, 2H), 4.61 (s, 2H), 4.54 (s, 2H), 4.49 (t, *J* = 4.2, 2H), 4.17 (d, *J* = 3.3, 2H), 1.33 - 1.23 (m, 3H), 1.12 (ds, 18H).

### EXAMPLE 8: Compound (XII): R¹= -CH₂CH₂-; R²= -CH₂OCH₂-; R³=-CH=CH-; Q= OH; PG= Si(ⁱPr)₃ 1-(2-Hydroxyethyl)-5-[(E)-(2-phenyl)vinyl]-4-[2-oxa-3-(4-triisopropylsilyloxyphenyl)-proyl]-1,2,3-triazole

A suspension of 1-(2-hydroxyethyl)-5-iodo-4-[2-oxa-3-(4-triisopropylsilyloxyphenyl)-proyl]-1,2,3-triazole (270 mg, 0.52 mmol) prepared in Example 7, E-2-phenylvinylboronic acid (119.8 mg, 0.79 mmol), bis(triphenylphosphine) palladium (II) dichloride (14.7 mg, 0.02 mmol) and potassium hydroxide (58.8 mg, 1.05 mmol) were dissolved in anhydrous THF (4 mL) and kept at 75ºC during 2 h. The product was purified by column chromatography (silica gel, EtOAc/hexanes 1:1). Yield: 330 mg (92%). ¹H NMR (500 MHz, CDCl₃) δ 7.49 (d, *J* = 7.2, 2H), 7.41 (t, *J* = 7.3, 2H), 7.39 - 7.34 (m, 1 H), 7.31 (d, *J* = 13.2, 1 H), 7.26 (d, *J* = 8.4, 2H), 6.98 (d, *J* = 16.3, 1H), 6.86 (d, *J* = 8.4, 2H), 4.72 (s, 2H), 4.58 (s, 2H), 4.51 - 4.46 (m, 2H), 4.18 (dd, *J* = 10.2, 5.4, 2H), 1.32 - 1.22 (m, 3H), 1.11 (d, *J* = 7.4, 18H).

### EXAMPLE 9: Compound (XII): R¹ = -[CH₂CH₂O]₃CH₂CH₂HN(CO)OCH₂CH₂-; R²= -CH₂OCH₂-; R³= -CH=CH-; Q= N₃; PG= Si(ⁱPr)₃ 1-(16-Azido-5-aza-3,8,11,14-tetraoxa-4-oxohexadecyl)-5-[(E)-(2-phenyl)vinyl]-4-[2-oxa-3-(4-triisopropylsilyloxyphenyl)-proyl]-1,2,3-triazole

In a flame-dried flask, 1-(2-hydroxyethyl)-5-[(E)-(2-phenyl)vinyl]-4-[2-oxa-3-(4-triisopropylsilyloxyphenyl)-proyl]-1,2,3-triazole (0.20 mmol, 100 mg) prepared as shown in Example 7, was dissolved in dry THF (1.5 mL) under nitrogen atmosphere and after addition of DIPEA (0.39 mmol, 69 µL), the mixture was cooled to 0ºC. Subsequently, a solution of triphosgene (0.12 mmol, 36 mg) was added dropwise and then the mixture was allowed to reach the room temperature during 30 minutes. The suspension was filtered through a celite pad and the solvent was evaporated under pressure to obtain the intermediate chloroformate [¹H NMR (500 MHz, CDCl₃) δ 7.50 (d, *J* = 7.3, 2H), 7.45 - 7.35 (m, 4H), 7.26 (d, *J* = 8.0, 2H), 6.92 (d, *J =* 16.4, 1 H), 6.86 (d, *J* = 7.9, 2H), 4.80-4.70 (m, 6H), 4.59 (s, 2H), 1.31 - 1.21 (m, 3H), 1.11 (d, *J* = 7.3, 18H)]. The product was dissolved in dry CH₂Cl₂ (1.5 mL) and sequentially DIPEA (0.39 mmol, 69 µL) and tetraethylene glycol (0.20 mmol, 43 mg) were added and the mixture was kept stirring overnight. The product was purified by column chromatography using CH₂Cl₂/MeOH 90/10 as eluent. Yield: 116 mg (78%). IR (cm⁻¹): 2944, 2866, 2102 (N₃), 1720 (C=O), 1509. ¹H NMR (500 MHz, CDCl₃) δ 7.47 (d, *J* = 7.7, 2H), 7.42 - 7.21 (m, 6H), 6.92 (d, *J =* 16.3, 1 H), 6.85 (d, *J* = 8.0, 2H), 4.72 (s, 2H), 4.64 (t, *J* = 4.8, 2H), 4.57 (s, 2H), 4.48 (s, 2H), 3.69 - 3.49 (m, 11 H), 3.45 - 3.41 (m, 2H), 3.39 - 3.32 (m, 2H), 3.28 (t, *J* = 10.9, 2H), 1.30 - 1.19 (m, 3H), 1.09 (d, *J* = 7.4, 18H).

### EXAMPLE 10: Compound (I): R¹= -CH₂CH₂-; R²= -CH₂OCH₂-; R³=-CH=CH-; X= N₃; Y= p-C₆H₄OH; Z= Ph 1-(2-Azidoethyl)-5-[(E)-(2-phenyl)vinyl]-4-[2-oxa-3-(4-triisopropylsilyloxyphenyl)-proyl]-1,2,3-triazole

In a dried flask cyanuric chloride (55.93 mg, 0.30 mmol) and DMF (61.06 µL) were warmed at 25 °C for 10 min. After the formation of a white solid, CH₂Cl₂ (0.5 mL) was added, followed by 1-(2-hydroxyethyl)-5-[(E)-(2-phenyl)vinyl]-4-[2-oxa-3-(4-triisopropylsilyloxyphenyl)-proyl]-1,2,3-triazole (70 mg, 0.14 mmol). The mixture was kept at room temperature during one hour and the solvent was evaporated. The resulting crude product was dissolved again in acetone, sodium azide (89.71 mg, 1.38 mmol) was added and the mixture was stirred for 24 hours. Then, cesium fluoride (300 mg, 2.0 mmol) was added, and the resulting crude was evaporated and purified by column chromatography (silica gel, EtOAc/hexanes 1:1). ¹H NMR (500 MHz, CDCl₃) δ 7.50 (d, *J* = 7.2, 2H), 7.47 - 7.33 (m, 4H), 7.33 - 7.25 (m, 3H), 6.95 (d, *J* = 16.3, 1 H), 6.87 (d, *J* = 8.2, 2H), 4.75 (s, 2H), 4.71 (t, *J* = 6.4, 2H), 4.60 (s, 2H), 4.01 (t, *J* = 6.4, 2H).

### EXAMPLE 11: Compound (I): R¹= -[CH₂CH₂O]₃CH₂CH₂HN(CO)OCH₂CH₂-; R²= -CH₂OCH₂-; R³ = -CH=CH-; X= N₃; Y= p-C₆H₄OH; Z= Ph 1-(16-Azido-5-aza-3,8,11,14-tetraoxa-4-oxohexadecyl)-5-[(E)-(2-phenyl)vinyl]-4-[2-oxa-3-(4-hydroxyphenyl)-proyl]-1,2,3-triazole

A suspension of 1-(16-azido-5-aza-3,8,11,14-tetraoxa-4-oxohexadecyl)-5-[(E)-(2-phenyl)vinyl]-4-[2-oxa-3-(4-triisopropylsilyloxyphenyl)-proyl]-1,2,3-triazole (0.14 mmol, 105 mg) prepared in Example 9, and cesium fluoride (0.70 mmol, 106 mg) in methanol (1.5 mL) was stirred at room temperature for one hour. Then the solvent was evaporated and the product was purified by column chromatography using CH₂Cl₂/MeOH 95:5 as eluent. Yield: 50 mg (60%). IR(cm⁻¹): 3328, 2867, 2101 (N₃), 1705 (C=O), 1517. ¹H NMR (500 MHz, CDCl₃) δ 7.47 (d, *J* = 7.4, 2H), 7.44 - 7.25 (m, 5H), 7.20 (dd, *J* = 17.2, 8.0, 2H), 6.86 (d, *J* = 16.6, 1 H), 6.80 (d, *J* = 8.1, 2H), 4.75 (s, 2H), 4.64 (d, *J* = 4.8, 2H), 4.57 (s, 2H), 4.47 (s, 2H), 3.58 (ddd, *J =* 35.4, 14.6, 6.2, 10H), 3.39 - 3.35 (m, 2H), 3.35 - 3.27 (m, 2H), 3.21 (d, *J* = 4.8, 2H).

### EXAMPLE 12: Poly(etheretherketone) PEEK with the surface modified as cycloalkyne. Material according to formula [PEEK]=N-O-(R⁴)-X, wherein: R¹= -CH₂CH₂OCH₂CH₂O-; X= cyclooctyn-3-yl

Compact PEEK consisted of mechanically polished PEEK-1000 semicrystalline 20 x 20 x 5 mm size square samples from Ketron (KETRON PEEK 1000, ref. 41300000, PoliFluor S.L) and medical grade implantable PEEK CLASSIX LSG compact disk samples from Invibio (PoliFluor S.L). Porous PEEK consisted of disk samples (9 mm diameter/ 3 mm thin), prepared according to patent EP10382243.3 (Porous PEEK article as an implant) with VESTAKEEP^{®} 2000 P powder

Compact PEEK and porous PEEK materials with the surface modified as oxime [PEEK]=N-OH were prepared following a procedure described in Macromolecules, 1991, 24: 3045-3049.
PEEK oxime: Both compact and porous PEEK samples were cleaned by immersion in an ultrasonic bath with methanol for 30 min and subsequently dried at room temperature under reduced pressure overnight. Each set of 10 samples of porous and compact PEEK, 3.0 g of hydroxylamine hydrochloride, 10 mL of ethanol, and 2 mL water were introduced to a round-bottomed flask. Sodium hydroxide (5.5 g) was added in five portions, shaking after each addition. The balloon flask was purged with nitrogen, then heated at 40ºC for 24h, and finally refluxed for 24h. After cooling the suspenssion, the samples were extracted, rinsed successively with 10% aqueous HCl (5 x 30 mL) and ethanol (3 x 30 mL), and dried at room temperature.

3-(6-lodo-1,3-dioxahexyl)-cyclooctyne: To a solution of O-(cyclooctyn-3-yl)-diethylene glycol (100 mg, 0.47 mmol) in anhydrous DMF (10 mL) was added (PhO)₃PMel (0.43 g, 0.94 mmol). The resulting solution was stirred at r. t. for 30 min. Then, MeOH (1 mL) was added the mixture was evaporated at reduced pressure. The crude obtained was used without additional purification. Yield: 103.2 mg, (65%). ¹H-NMR (500 MHz, CDCl₃): δ 4.24 (t, 1 H, -CH-C=C-), 3.77-3.67 (m, 5H, O-CH₂), 3.53 (m, 1 H, -CH-O), 1.91 (t, 2H, CH₂-I), 2.25-1.45 (m, 10H, -CH₂-).
Compact and porous PEEK oxime samples (n=2), prepared as above, were introduced under nitrogen atmosphere into a test tube containing a mixture of potassium carbonate (0.41 g), 3-(6-lodo-1,3-dioxahexyl)-cyclooctyne (0.20 g) and acetone (2 mL). The suspension was stirred at 40ºC for 24 h., and the samples were washed repeatedly with water and methanol. Finally, the samples were dried at r.t. under vacuum for 5 h.
Surface characterization data for compact PEEK modified as cycloalkyne. XPS analysis: C 83.3%, O 14.8%, N 1.9%.
Surface characterization data for porous PEEK modified as cycloalkyne. Contact angle: 139.2º ±18.0. XPS analysis: C 81.8%, O 17.2%, N 1.0%.

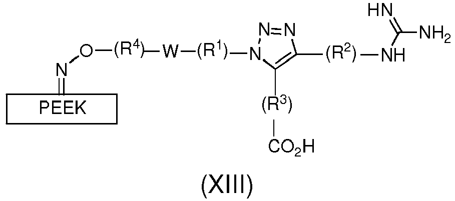

### EXAMPLE 13: Porous PEEK with the surface modified as RGD mimetic. Material (XIII): R¹=R³= -CH₂CH₂-; R²= -CH₂OCH₂CH₂-; R⁴=-CH₂CH₂OCH₂CH₂O-; W= 4,5-bicyclooctene-1,2,3-triazole

Porous PEEK oxime samples (n=2) functionalized as cycloalkyne as described in Example 12, were introduced under nitrogen atmosphere into a test tube containing a 2 mL of a THF/H₂O 1:1 mixture. Then, 0.2 mg of compound of formula (I): (R¹= R³= -CH₂CH₂-; R²= -CH₂OCH₂CH₂-; X= N₃; Y= -NHC(=NH)NH₂; Z= CO₂H), prepared in Example 5, were added and the suspension was stirred at 40ºC for 24 h. The sample was washed repeatedly with a solution of HCl 0.1 M, an aqueous solution of NH₃ (pH=11), water and methanol and dried at room temperature under vacuum for 5 h.
Surface XPS analysis for porous PEEK modified with RGD mimetic: C 79.4%, O 18.7% N 1.9%).

### EXAMPLE 14: Compact PEEK with the surface modified as RGD mimetic. Material (XIII): R¹=R³= -CH₂CH₂-; R²= -CH₂OCH₂CH₂-; R⁴=-CH₂CH₂OCH₂CH₂O-; W= 4,5-bicyclooctene-1,2,3-triazole

Compact PEEK-M oxime samples (n=2) functionalized as cycloalkyne as described in Example 12, was reacted with 0.2 mg of RGD mimetic of formula (I): (R¹= R³= -CH₂CH₂-; R²= -CH₂OCH₂CH₂-; X= N₃; Y= -NHC(=NH)NH₂; Z= CO₂H). Reaction conditions and purification were identical to the Example 13. Surface characterization data for polished compact PEEK modified with RGD mimetic. XPS analysis: C 84.4%, O 13.3%, N 2.3%.

### EXAMPLE 15: Compact PEEK-M with the surface modified as RGD mimetic. Material (XIII): R¹=R³= -CH₂CH₂-; R²= -CH₂OCH₂CH₂-; R⁴= - [CH₂CH₂O]₃CH₂CH₂HN(CO)OCH₂CH₂-; W= 4,5-bicyclooctene-1,2,3-triazole

Compact PEEK oxime samples (n=2) functionalized as cycloalkyne as described in Example 12, was reacted with 0.2 mg of RGD mimetic of formula (I): (R¹= -[CH₂CH₂O]₃CH₂CH₂HN(CO)OCH₂CH₂-; R²= -CH₂OCH₂CH₂-; R³= -CH₂CH₂-; X= N₃; Y= -NHC(=NH)NH₂; Z= CO₂H prepared as described in Example 6). Reaction conditions and purification were identical to the Example 13.

Surface characterization data for polished compact PEEK modified with RGD mimetic. XPS analysis: C 82.2%, O 14.6%, N 3.2%.

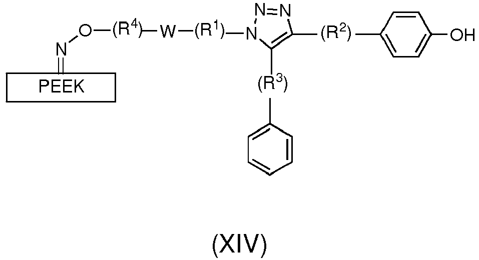

### EXAMPLE 16: Compact PEEK with the surface modified as OGP mimetic. Material XIV): R¹= -CH₂CH₂-; R²= -CH₂OCH₂-; R³= -CH=CH-; R⁴= - CH₂CH₂CH₂CH₂O-; W= 4,5-bicyclooctene-1,2,3-triazole

Compact PEEK oxime samples (n=2) functionalized as cycloalkyne as described in Example 12, was reacted with 0.2 mg of OGP mimetic of formula (I): (R¹= -CH₂CH₂-; R²= -CH₂OCH₂-; R³= -CH=CH-; X= N₃; Y= p-C₆H₄OH; Z= Ph prepared as described in Example 10). Reaction conditions and purification was identical to the Example 12.
Surface characterization data for polished compact PEEK modified with OGP mimetic. XPS analysis: C 75.6%, O 21.2%, N 3.2%.

### EXAMPLE 17: Compact PEEK-M with the surface modified with OGP mimetic. Material (XIV): R¹= -[CH₂CH₂O]₃CH₂CH₂HN(CO)OCH₂CH₂-; R²=-CH₂OCH₂-; R³= -CH=CH-; R⁴= -CH₂CH₂OCH₂CH₂O-; W= 4,5-bicyclooctene-1,2,3-triazole

A compact PEEK oxime discs functionalized as cycloalkyne as described in Example 12, was introduced under nitrogen atmosphere into a test tube containing 1 mL of THF/H₂O 1:1 mixture, 0.1 mg of OGP mimetic (I) (R¹=-[CH₂CH₂O]₃CH₂CH₂HN(CO)OCH₂CH₂-; R²= -CH₂OCH₂-; R³= -CH=CH-; X= N₃; Y= p-C₆H₄OH; Z= Ph) prepared as described in Example 11. The suspension was stirred at 40ºC for 24 h. The discs were washed repeatedly with THF, water and methanol and dried at room temperature under vacuum for 5 h.
Surface XPS analysis for the compact PEEK modified with OGP mimetic: C 79.8%, O 17.7% N 2.5%.

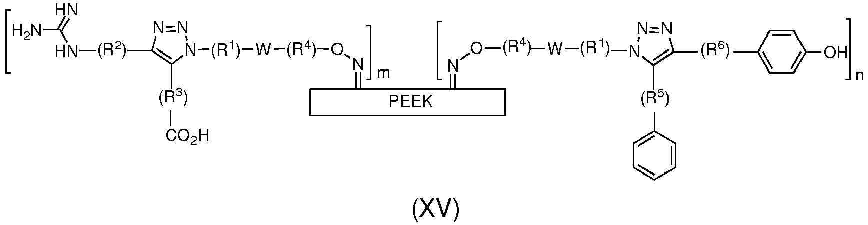

### EXAMPLE 18: Compact PEEK with the surface modified with a combination of RGD and OGP peptidomimetics. Material (XV): R¹=R³= -CH₂CH₂-; R²=-CH₂CH₂OCH₂-; R⁴= -OCH₂CH₂OCH₂CH₂-; R⁵= -CH=CH-; R⁶= -CH₂OCH₂-; W= 4,5-bicyclooctene-1,2,3-triazole

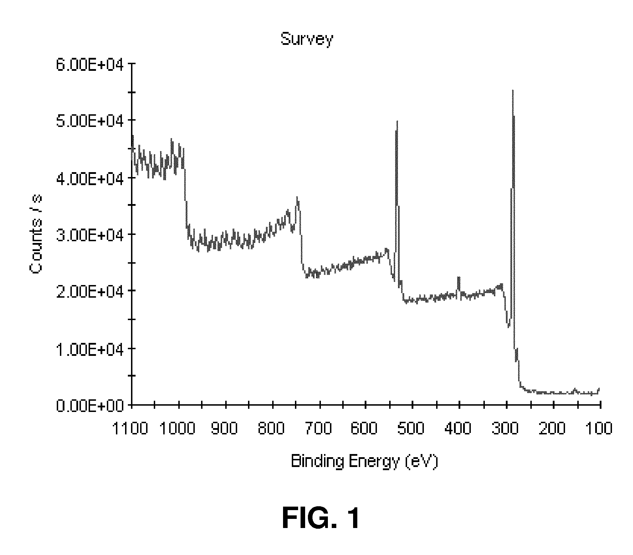

Compact PEEK oxime discs (n=2) functionalized as cycloalkyne as described in Example 12, were introduced under nitrogen atmosphere into a test tube containing 2 mL of THF/H₂O 1:1 mixture, 0.1 mg of RGD mimetic of formula (I) (R¹=R³= -CH₂CH₂-; R²= -CH₂OCH₂CH₂; X= N₃; Y= -NHC(=NH)NH₂; Z= CO₂H) prepared as described in Example 5, and 0.1 mg of OGP mimetic of formula (I) (R¹= -CH₂CH₂-; R²= -CH₂OCH₂-; R³= -CH=CH-; X= N₃; Y= p-C₆H₄OH; Z= Ph) prepared as described in Example 10. The suspension was stirred at 40ºC for 24 h. The discs were washed repeatedly with a solution of HCl 0.1 M, an aqueous solution of NH₃ (pH=11), water and methanol and dried at room temperature under vacuum for 5 h.
Surface XPS analysis for compact PEEK (XV) modified with RGD and OGP: C 74.9%, O 21.6% N 3.59% (Figure 1).

### EXAMPLE 19: Compact PEEK with the surface modified with a combination of RGD and OGP peptidomimetics. Material (XV): R¹=-[CH₂CH₂O]₃CH₂CH₂HN(CO)OCH₂CH₂-; R³= -CH=CH-; R⁴=-OCH₂CH₂OCH₂CH₂-; R⁵= -CH=CH-; R⁶= -CH₂OCH₂-; W= 4,5-bicyclooctene-1,2,3-triazole

Compact PEEK oxime discs (n=2) functionalized as cycloalkyne prepared as described in Example 12, were introduced under nitrogen atmosphere into a test tube containing 2 mL of THF/H₂O 1:1 mixture, 0.1 mg of RGD mimetic of formula (I) (R¹ = -[CH₂CH₂O]₃CH₂CH₂HN(CO)OCH₂CH₂-; R²= - CH₂OCH₂CH₂-; R³= -CH₂CH₂-; X= N₃; Y= -NHC(=NH)NH₂; Z= CO₂H) prepared as described in Example 10, and 0.1 mg of OGP mimetic (I) (R¹ =-[CH₂CH₂O]₃CH₂CH₂HN(CO)OCH₂CH₂-; R²= -CH₂OCH₂-; R³= -CH=CH-; X= N₃; Y= p-C₆H₄OH; Z= Ph) prepared as described in Example 11. The suspension was stirred at 40ºC for 24 h. The discs were washed repeatedly with a solution of HCl 0.1 M, an aqueous solution of NH₃ (pH=11), water and methanol and dried at room temperature under vacuum for 5 h

## Claims

1. A 1,4,5-trisubstituted 1,2,3-triazole compound of formula (I): 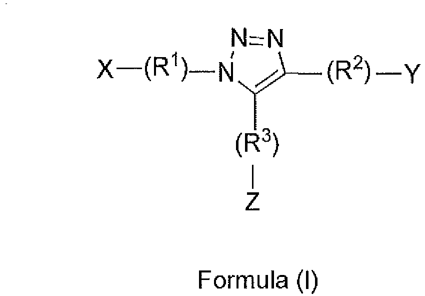 wherein:
R¹ is a biradical selected from the group consisting of C₁₋₂₀ alkylene; in which 0, 1, 2, 3, 4, 5 or 6 -CH₂- groups are optionally replaced by groups selected from -O-, -S-, -C(O)O-, -C(O)NH-, -C(O)N(C₁₋₄alkyl)-, -NHC(O)NH-,-NHC(O)O-; and
R² is a biradical independently selected from the group consisting of: C₁₋₆ alkylene; in which 0, 1, 2 or 3 -CH₂- groups are optionally replaced by groups selected from -O- and -S-; optionally substituted with one or more groups selected from C₁₋₄ alkyl, phenyl, C₆₋₁₀ aryl; and
R³ is a biradical selected from the group consisting of: C₁₋₆ alkylene; optionally containing one or more -C=C- bonds; optionally containing -C=C- bonds; in which 0, 1, 2 or 3 -CH₂- groups are optionally replaced by groups selected from -O- and -S-; optionally substituted with one or more groups selected from C₁₋₄ alkyl, phenyl, -F, -Cl, -OH, -O(C₁₋₄alkyl), -S(C₁₋₄alkyl), -SO₂Ph,-CN, -NO₂, -CO(C₁₋₄alkyl), -CO₂H, -CO₂(C₁₋₄alkyl), -CONH₂, -CONH(C₁₋₄alkyl), -CON(C₁₋₄alkyl)₂; and
X is a group selected from the group consisting of: azido, N-maleimido, N-maleimido-furan cycloadduct, thiol, thio acid, azido, sulfonylazido, ethynyl, iodoethynyl and an activated cyclooctynyl group represented by the formulae: 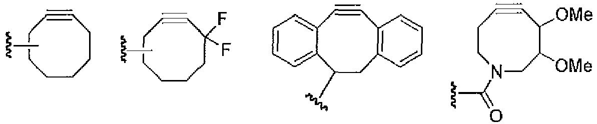
Y is a group selected from -NHC(=NH)NH₂ and C₆H₄-OH
Z is a group selected from -CO₂H and Ph, and
when Y is -NHC(=NH)NH₂, Z is -CO₂H, and
when Y is C₆H₄-OH, Z is Ph.

2. The compound according to claim 1 wherein R¹ is selected from the group consisting of -(CH₂CH₂O)rCH₂CH₂-; where r is an integer between 0 and 8, R² is selected from the group consisting of -CH₂OCH₂CH₂- or -CH₂OCH₂-, and R³ is selected from the group consisting of -CH₂CH₂- or -CH=CH-.

3. The compound according to claim 1-2 wherein Y is -NHC(=NH)NH₂ and Z is -CO₂H.

4. The compound according to claim 1-2 wherein Y is C₆H₄-OH and Z is Ph.

5. The compound according claim 4 wherein Y is p-C₆H₄-OH and Z is Ph.

6. A process for preparing the compounds according to the claim 3, which comprises:
a) reacting an azide of formula Q-(R¹)-N₃ (II) with an alkyne of formula T-C≡C-(R²)-NH-PG (protecting group) (III) to obtain a triazole of formula (IV),
b) reacting a triazole of the formula (IV) with a compound of formula S-(R³)-CO₂R^{a} (V) or an alkene of formula H₂C=CH-CO₂R^{a} (VI), to provide a compound of formula (VII),
c) replacing the Q group in the compounds of formula (VII) by a group X, and
d) removing the protecting group PG in compounds of the formula (VIII) and e) reacting the intermediate amines with H₂NC(=NH)SO₃H. 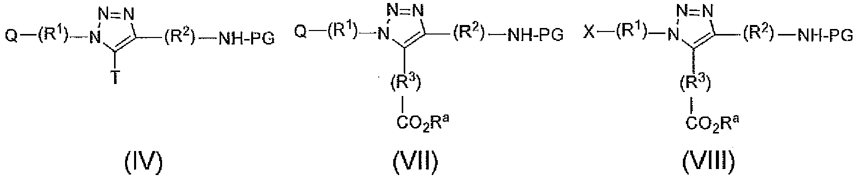 wherein:
R¹, R², R³ and X are as defined in claim 1; R^{a} is H, methyl, ethyl, tert-butyl or benzyl; T is H or I; Q is HO, methanesulfonyloxy, p-toluenesulfonyloxy, 2-nitrobenzenesulfonyloxy, 4-nitrobenzenesulfonyloxy, trifluoromethanesufonyloxy, Cl, Br or I; PG is H, tert-butoxycarbonyl, allyloxycarbonyl or benzyloxycarbonyl; S in step b) is B(OH)₂, methanesulfonyloxy, p-toluenesulfonyloxy, 2-nitrobenzenesulfonyloxy, 4-nitrobenzenesulfonyloxy, trifluoromethanesufonyloxy, Cl, Br or I.

7. A process for preparing the compounds (I), according to the claim 4-5, which comprises:
a) reacting an azide of formula Q-(R¹)-N₃ (II) with an alkyne of formula T-C≡C-(R²)-(C₆H₄OH)-PG (IX) to obtain a triazole of formula (X),
b) reacting a triazole of the formula (X) with a compound of formula S-(R³)-Ph (XI), to provide a compound of formula (XII),
c) replacing the Q group in the compounds of formula (XII) by a group X, and
d) removing the protecting group PG. 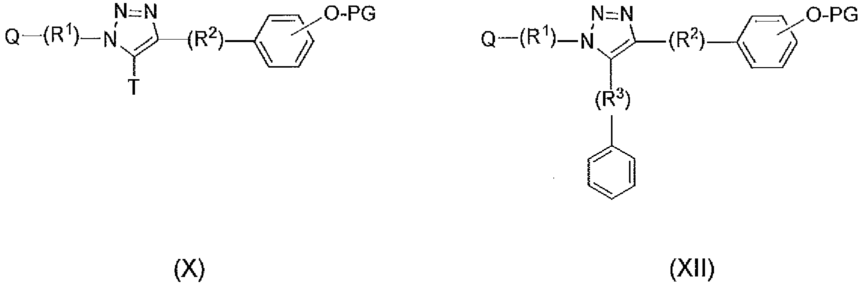 wherein:
R¹, R², R³ and X are as defined in claim 2; T is H or I; Q is HO, methanesulfonyloxy, p-toluenesulfonyloxy, 2-nitrobenzenesulfonyloxy, 4-nitrobenzenesulfonyloxy, trifluoromethanesufonyloxy, Cl, Br or I; PG is H, tert-butoxycarbonyl, allyloxycarbonyl or benzyloxycarbonyl; S in step b) is B(OH)₂; PG is Si(i-Pr)₃.

8. A material with the surface chemically modified wherein the X group of the 1,4,5-trisubstituted 1,2,3-triazole compound according claim 1 to 5 is replaced by a W group represented by the formulae.

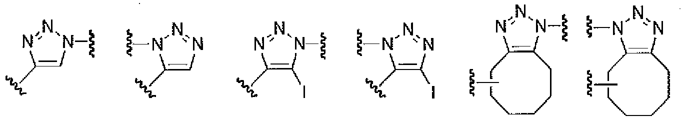

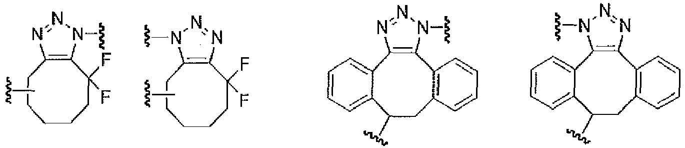

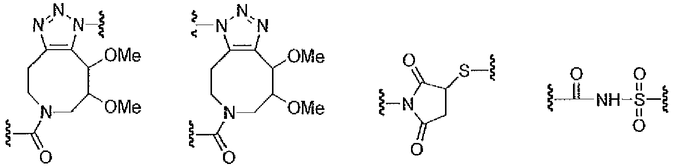

9. The material with the surface chemically modified according claim 8 wherein the 1,4,5-trisubstituted 1,2,3-triazole compound is defined according claim 3.

10. The material with the surface chemically modified according claim 8 wherein the 1,4,5-trisubstituted 1,2,3-triazole compound is defined according claim 5.

11. Medical device wherein the device is made from the material as defined in claim 8-10.

12. Medical device according claim 11 wherein the medical device is an endosseous implant, or tissue engineering scaffold or cell culture matrix, suitable for the replacement or regeneration of human and animal organs.

## Patentansprüche

1. 1,4,5-trisubstituierte 1,2,3-Triazolverbindung der Formel (I): 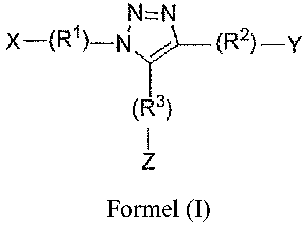 worin
R¹ ein Biradikal ist, ausgewählt aus der Gruppe bestehend aus C₁₋₂₀ Alkylen; worin 0, 1, 2, 3, 4, 5 oder 6 -CH₂- Gruppen gegebenenfalls durch Gruppen ausgewählt aus -O-, -S-, -C(O)O-,-C(O)NH-, -C(O)N(C₁₋₄Alkyl)-, -NHC(O)NH-, -NHC(O)O- ersetzt sind; und
R² ein Biradikal ist, unabhängig voneinander ausgewählt aus der Gruppe bestehend aus:
Alkylen; worin 0, 1, 2 oder 3 -CH₂- Gruppen gegebenenfalls durch Gruppen ausgewählt aus - O- und -S- ersetzt sind; gegebenenfalls substituiert mit einer oder mehreren Gruppen ausgewählt aus Alkyl, Phenyl, Aryl; und
R³ ein Biradikal ist, ausgewählt aus der Gruppe bestehend aus: C₁₋₆ Alkylen; gegebenenfalls enthaltend eine oder mehrere -C=C- Bindungen; gegebenenfalls enthaltend -C=C- Bindungen;
worin 0, 1, 2 oder 3 -CH₂- Gruppen gegebenenfalls durch Gruppen ausgewählt aus -O- und - S- ersetzt sind; gegebenenfalls substituiert mit einer oder mehreren Gruppen ausgewählt aus C₁₋₄ Alkyl, Phenyl, -F, -CI, -OH, -O(C₁₋₄ Alkyl), -S(C₁₋₄ Alkyl), -SO₂Ph, -CN, -NO₂, -CO(C₁₋₄ Alkyl), -CO₂H, -CO₂(C₁₋₄ Alkyl), -CONH₂, -CONH(C₁₋₄ Alkyl), -CON(C₁₋₄ Alkyl)₂; und
X eine Gruppe ist, ausgewählt aus der Gruppe bestehend aus: Azido, N-Maleimido, N-Maleimidofuran-Cycloaddukt, Thiol, Thiosäure, Azido, Sulfonylazido, Ethinyl, Jodethinyl und einer aktivierten Cyclooctinylgruppe dargestellt durch die Formeln: 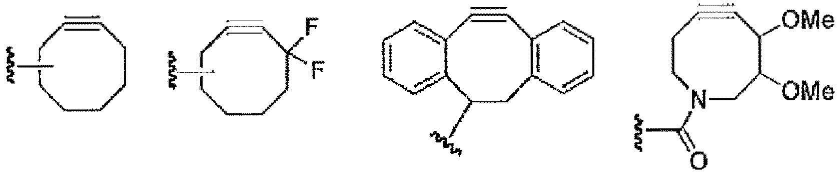
Y eine Gruppe ist, die ausgewählt ist aus -NHC(=NH)NH₂ und C₆H₄-OH
Z eine Gruppe ist, die ausgewählt ist aus -CO₂H und Ph, und
wenn Y -NHC(=NH)NH₂ ist, ist Z -CO₂H, und
wenn Y C₆H₄-OH ist, ist Z Ph.

2. Verbindung nach Anspruch 1, worin R¹ ausgewählt ist aus der Gruppe bestehend aus - (CH₂CH₂O)rCH₂CH₂-; wobei r eine ganze Zahl zwischen 0 und 8 ist, R² ausgewählt ist aus der Gruppe bestehend aus -CH₂OCH₂CH₂- oder -CH₂OCH₂- und R³ ausgewählt ist aus der Gruppe bestehend aus -CH₂CH₂- oder -CH=CH-.

3. Verbindung nach Anspruch 1-2 worin Y -NHC(=NH)NH₂ ist und Z -CO₂H ist.

4. Verbindung nach Anspruch 1-2 worin Y C₆H₄-OH ist und Z Ph ist.

5. Verbindung nach Anspruch 4 worin Y p-C₆H₄-OH ist und Z Ph ist.

6. Verfahren zur Herstellung der Verbindungen nach Anspruch 3, das umfasst:
a) Reagieren eines Azids der Formel Q-(R¹)-N₃ (II) mit einem Alkin der Formel T-C≡C-(R²)-NH-PG (Schutzgruppe) (III) um ein Triazol der Formel (IV) zu erhalten,
b) Reagieren eines Triazols der Formel (IV) mit einer Verbindung der Formel S-(R³)-CO₂R^{a} (V) oder einem Alken der Formel H₂C=CH-CO₂R^{a} (VI), um eine Verbindung der Formel (VII) zur Verfügung zu stellen,
c) Ersetzen der Q Gruppe in den Verbindungen der Formel (VII) durch eine Gruppe X, und
d) Entfernen der Schutzgruppe PG in Verbindungen der Formel (VIII) und
e) Reagieren der Zwischenprodukt-Amine mit H₂NC(=NH)SO₃H. 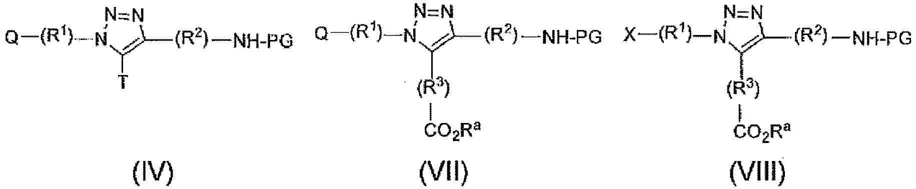 worin:
R¹, R², R³ und X wie in Anspruch 1 definiert sind; R^{a} ist H, Methyl, Ethyl, tert-Butyl oder
Benzyl; T ist H oder J; Q ist HO, Methansulfonyloxy, p-Toluolsulfonyloxy, 2-Nitrobenzensulfonyloxy, 4-Nitrobenzensulfonyloxy, Trifluormethansulfonyloxy, Cl, Br oder J; PG ist H, tert-Butoxycarbonyl, Allyloxycarbonyl oder Benzyloxycarbonyl; S in Schritt b) ist B(OH)₂, Methansulfonyloxy, p-Toluolsulfonyloxy, 2-Nitrobenzensulfonyloxy, 4-Nitrobenzensulfonyloxy, Trifluormethansulfonyloxy, Cl, Br oder J.

7. Verfahren zur Herstellung der Verbindungen (I) nach den Ansprüchen 4-5, das umfasst:
a) Reagieren eines Azids der Formel Q-(R¹)-N₃ (II) mit einem Alkin der Formel T-C≡C-(R²)-(C₆H₄OH)-PG (IX), um ein Triazol der Formel (X) zu erhalten,
b) Reagieren eines Triazols der Formel (X) mit einer Verbindung der Formel S-(R³)-Ph (XI), um eine Verbindung der Formel (XII) zur Verfügung zu stellen,
c) Ersetzen der Gruppe Q in den Verbindungen der Formel (XII) durch eine Gruppe X, und
d) Entfernen der Schutzgruppe PG. 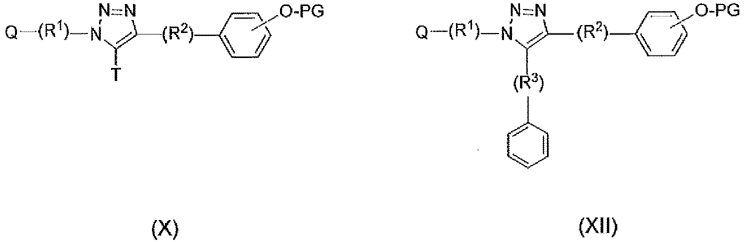 worin:
R¹, R², R³ und X wie in Anspruch 2 definiert sind; T ist H oder J; Q ist HO, Methansulfonyloxy, p-Toluolsulfonyloxy, 2-Nitrobenzensulfonyloxy, 4-Nitrobenzensulfonyloxy, Trifluormethansulfonyloxy, Cl, Br oder J; PG ist H, tert-Butoxycarbonyl, Allyloxycarbonyl oder Benzyloxycarbonyl; S in Schritt b) ist B(OH)₂; PG ist Si(i-Pr)₃.

8. Material mit einer chemisch modifizierten Oberfläche, wobei die Gruppe X der 1,4,5-trisubstituierten 1,2,3-Triazolverbindung nach Anspruch 1 bis 5 durch eine Gruppe W ersetzt ist, die dargestellt wird durch die Formeln

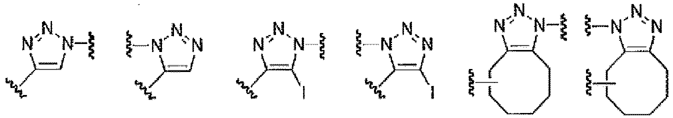

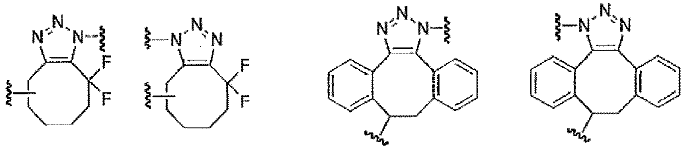

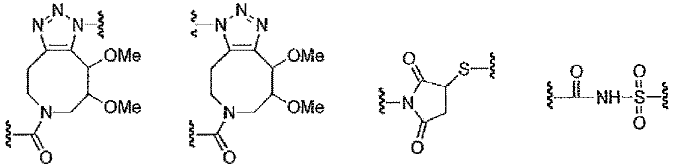

9. Material mit einer chemisch modifizierten Oberfläche nach Anspruch 8, wobei die 1,4,5-trisubstituierte 1,2,3-Triazolverbindung wie gemäß Anspruch 3 definiert ist.

10. Material mit einer chemisch modifizierten Oberfläche nach Anspruch 8, wobei die 1,4,5-trisubstituierte 1,2,3-Triazolverbindung wie gemäß Anspruch 5 definiert ist.

11. Medizinische Vorrichtung, wobei die Vorrichtung aus dem wie in Anspruch 8-10 definierten Material hergestellt ist.

12. Medizinische Vorrichtung nach Anspruch 11, wobei die medizinische Vorrichtung ein enossales Implantat oder ein Gewebe-Entwicklungsgerüst oder eine Zellkulturmatrix ist, geeignet für den Ersatz oder die Regeneration von menschlichen und tierischen Organen.

## Revendications

1. Composé 1,2,3-triazole 1,4,5-trisubstitué de formule (I) : 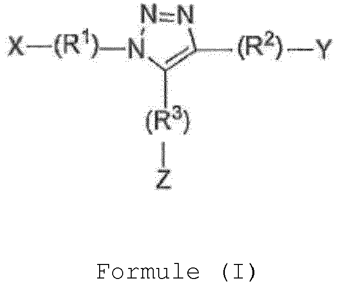 dans laquelle :
R¹ est un biradical choisi dans le groupe consistant en un alkylène en C₁₋₂₀ ; dans lequel 0, 1, 2, 3, 4, 5 ou 6 groupes -CH₂- sont optionnellement remplacés par des groupes choisis dans -0-, -S-,-C(O)O-, -C(O)NH-, -C(O)N(alkyle en C₁₋₄) -, NHC(O)NH-,-NHC(O)O- ; et
R² est un biradical indépendamment choisi dans le groupe consistant en un alkylène en C₁₋₆ ; dans lequel 0, 1, 2 ou 3 groupes -CH₂- sont optionnellement remplacés par des groupes choisis dans -0- et -S- ; optionnellement substitués par un ou plusieurs groupes choisis dans un alkyle en C₁₋₄, un phényle, un aryle en C₆₋₁₀ ; et
R³ est un biradical choisi dans le groupe consistant en un alkylène en C₁₋₆ ; contenant optionnellement une ou plusieurs liaisons -C=C- ; contenant optionnellement des liaisons -C≡C- ; dans lesquelles 0, 1, 2 ou 3 groupes -CH₂- sont optionnellement remplacés par des groupes choisis dans -0- et -S- ; optionnellement substitués par un ou plusieurs groupes choisis dans un alkyle en C₁₋₄, un phényle, -F, -Cl-, -OH, -O(alkyle en C₁₋₄), -S(alkyle en C₁₋₄), -SO₂Ph, -CN, -NO₂, -CO(alkyle en C₁₋₄), -CO₂H,-CO₂(alkyle en C₁₋₄), -CONH₂, -CONH(alkyle en C₁₋₄),-CON(alkyle en C₁₋₄)₂ ; et
X est un groupe choisi dans le groupe consistant en un azido, un N-maleimido, un cycloadduit de N-maleimido-furanne, un thiol, un thioacide, un azido, un sulfonylazido, un éthynyle, un iodoéthynyle et un groupe cyclooctynyle activé, représenté par les formules : 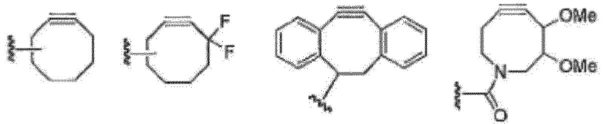
Y est un groupe choisi dans -NHC(=NH)NH₂ et C₆H₄-OH
Z est un groupe choisi dans -CO₂H et Ph, et
lorsque Y est -NHC(=NH)NH₂, Z est -CO₂H, et
lorsque Y est C₆H₄-OH, Z est Ph.

2. Composé selon la revendication 1, dans lequel R¹ est choisi dans le groupe consistant en-(CH₂CH₂O)rCH₂CH₂- ; où r est un entier compris entre 0 et 8, R² est choisi dans le groupe consistant en-CH₂OCH₂CH₂- ou -CH₂OCH₂-, et R³ est choisi dans le groupe consistant en -CH₂CH₂- ou -CH=CH-.

3. Composé selon les revendications 1 et 2, dans lequel Y est -NHC(=NH)NH₂ et Z est -CO₂H.

4. Composé selon les revendications 1 et 2, dans lequel Y est C₆H₄-OH et Z est Ph.

5. Composé selon la revendication 4, dans lequel Y est p-C₆H₄-OH et Z est Ph.

6. Procédé de préparation des composés selon la revendication 3, qui comprend :
a) la mise en réaction d'un azyde de formule Q-(R¹)-N³(II) avec un alcyne de formule T-C≡C-(R²)-NH-PG (groupe protecteur) (III) pour obtenir un triazole de formule (IV),
b) la mise en réaction d'un triazole de la formule (IV) avec un composé de formule S-(R³)-CO₂R^{a}(V) ou un alcène de formule H₂C=CH-CO₂R^{a}(VI), pour produire un composé de formule (VII),
c) le remplacement du groupe Q dans les composés de formule (VII) par un groupe X, et
d) l'élimination du groupe protecteur PG dans des composés de la formule (VIII) et e) la mise en réaction des amines intermédiaires avec H₂NC(=NH)SO₃H. 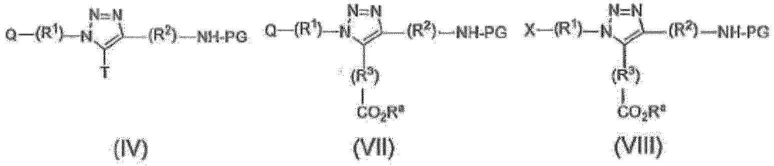 dans laquelle :
R¹, R², R³ et X sont tels que définis dans la revendication 1 ; R^{a} est H, un méthyle, un éthyle, un tert-butyle ou un benzyle ; T est H ou I ; Q est HO, un méthanesulfonyloxy, un p-toluènesulfonyloxy, un 2-nitrobenzènesulfonyloxy, un 4-nitrobenzènesulfonyloxy, un trifluorométhanesulfonyloxy, Cl, Br ou I ; PG est H, un tert-butoxycarbonyle, un allyloxycarbonyle, ou un benzyloxycarbonyle ; S à l'étape b) est B(OH)₂, un méthanesulfonyloxy, un p-toluènesulfonyloxy, un 2-nitrobenzènesulfonyloxy, un 4-nitrobenzènesulfonyloxy, un trifluorométhanesulfonyloxy, Cl, Br ou I ;

7. Procédé de préparation des composés (I) selon les revendications 4 et 5, qui comprend :
a) la mise en réaction d'un azyde de formule Q-(R¹)-N₃(II) avec un alcyne de formule T-C≡C-(R²)-(C₆H₄-OH)-PG) (IX) pour obtenir un triazole de formule (X),
b) la mise en réaction d'un triazole de la formule (X) avec un composé de formule S-(R³)-Ph (XI), pour produire un composé de formule (XII),
c) le remplacement du groupe Q dans les composés de formule (XII) par un groupe X, et
d) l'élimination du groupe protecteur PG. 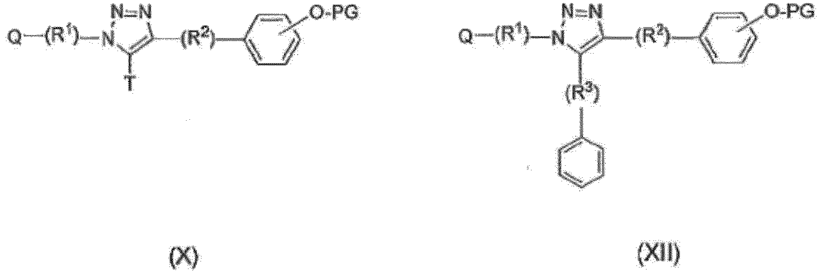 dans laquelle :
R¹, R², R³ et X sont tels que définis dans la revendication 2 ; T est H ou I, un méthanesulfonyloxy, un p-toluènesulfonyloxy, un 2-nitrobenzènesulfonyloxy, un 4-nitrobenzènesulfonyloxy, un trifluorométhanesulfonyloxy, Cl, Br ou I ; PG est H, un tert-butoxycarbonyle, un allyloxycarbonyle ou un benzyloxycarbonyle ; S à l'étape b) est B(OH)₂ ; PG est Si(i-Pr)₃.

8. Matériau avec la surface chimiquement modifiée, dans lequel le groupe X du composé 1,2,3-triazole 1,4,5-trisubstitué selon les revendications 1 à 5 est remplacé par un groupe W, représenté par les formules :

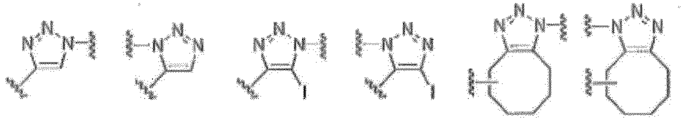

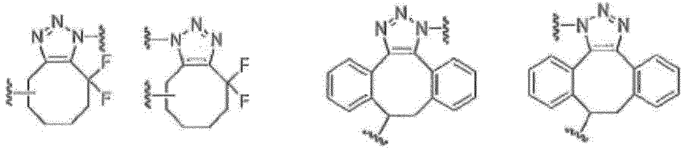

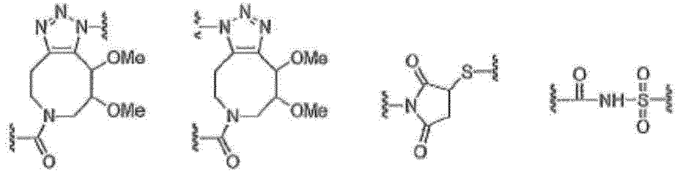

9. Matériau avec la surface chimiquement modifiée, selon la revendication 8, dans lequel le composé 1,2,3-triazole 1,4,5-trisubstitué est défini selon la revendication 3.

10. Matériau avec la surface chimiquement modifiée, selon la revendication 8, dans lequel le composé 1,2,3-triazole 1,4,5-trisubstitué est défini selon la revendication 5.

11. Dispositif médical, dans lequel le dispositif est réalisé dans le matériau, tel que défini dans les revendications 8 à 10.

12. Dispositif médical, selon la revendication 11, dans lequel le dispositif médical est un implant endo-osseux, un échafaudage d'ingénierie tissulaire ou une matrice pour culture cellulaire, conçu(e) pour remplacer ou régénérer des organes humains et animaux.
